# EUROPEAN PATENT SPECIFICATION

(11) **EP 3 732 277 B1**
(45) Date of publication and mention of the grant of the patent: **13.10.2021**
(21) Application number: 18814909.0
(22) Date of filing: 10.12.2018
(51) Int. Cl.: C11D 3/22, C11D 3/50, C11D 11/00, C11D 17/00, A61K 8/42, A61K 8/44, A61K 8/46, A61K 8/73, A61K 8/11, A61Q 13/00, A61Q 19/10

(54) **NON-SPHERICAL MICROCAPSULE**
ASPHÄRISCHE MIKROKAPSEL
MICROCAPSULE NON SPHÉRIQUE

(30) Priority: 29.12.2017 WO PCT/CN2017/119862; 15.02.2018 EP 18156845
(43) Date of publication of application: 04.11.2020
(73) Proprietor: Unilever Global IP Limited, Wirral, CH62 AZD (GB); Unilever IP Holdings B.V., 3013 AL Rotterdam (NL)
(72) Inventor: JIN, Huajin, Shanghai, 200335 (CN); PAN, Xiaoyun, Shanghai, 200335 (CN)
(74) Representative: Fijnvandraat, Arnoldus
(86) International application number: PCT/EP2018/084141
(87) International publication number: WO 2019/129477

(56) References cited:
- WO-A1-03/055314
- WO-A1-2006/007393
- WO-A1-2010/126742
- WO-A1-2017/175164
- DE-A1-102015 225 429

## Description

### Field of the invention

The present invention relates to non-spherical microcapsule. In particular, the present invention is related to a microcapsule comprising benefit agent and water-insolvable solid particle, wherein the microcapsule has an aspect ratio of from 3:1 to 12:1 and the weight ratio of the particle to the benefit agent is 1:5 to 50:1.

### Background of the invention

Many home care and personal care products seek to deliver benefit agents to substrates such as textiles, hard surfaces, hair and skin. To achieve a long-lasting benefit agent release performance, encapsulation of the benefit agent in microcapsule has been proposed as a means, in particular for the perfume. When applied, the microcapsules may be deposition onto the substrates, for example onto clothes, and broken by action of pressure and/or rubbing when consumers get dressed. The perfume is released and brings superior sensory to the consumers

WO 2010/126742 A1 discloses a cleaning composition comprising film flakes comprising embedded fragrance.

However, there is still much room for improvement from many aspects. One aspect is to improve the efficiency of the deposition of encapsulation microcapsules. Deposition aid is one way to improve the deposition efficiency. The use of deposition aids is however not always desired.

In addition, the ingredients in the home care or person care products, may affect the deposition efficiency of the microcapsules when the microcapsules are incorporated into the products. Therefore, we have recognized that there is a need to develop microcapsules with improved deposition efficiency onto skin even when they are included into the products. We developed microcapsule comprising benefit agent and water-insolvable solid particle, wherein the microcapsule has an aspect ratio of from 3:1 to 12:1 and the weight ratio of the particle to the benefit agent is 1:5 to 50:1. It was surprisingly found that the such microcapsules have improved deposition efficiency even when they were included into skin cleansing composition.

### Summary of the invention

In a first aspect, the present invention is directed to a microcapsule comprising benefit agent and water-insolvable solid particle, wherein the microcapsule has an aspect ratio of from 3:1 to 12:1 and the weight ratio of the particle to the benefit agent is 1:5 to 50:1, where aspect ratio means the ratio of the length to width of the microcapsule as measured by optical microscopy, where length refers to the longest measurable distance of the microcapsule in the optical image and width refers to the longest measurable distance of the microcapsule along the direction perpendicular to the length of the microcapsule in the optical image, and where water-insolvable refers to a material that dissolves in water to give a solution with a concentration of less than 1 gram per litre, wherein,
the water-insolvable solid particle comprises a plurality of pores throughout the particles and the benefit agent is encapsulated inside the pores; wherein the particle comprises cellulose; wherein the benefit agent is fragrance, pro-fragrance, or a mixture thereof.

In a second aspect, the present invention is directed to a process for preparing microcapsule of the present invention comprising steps of (a) dissolving the water-insolvable solid and benefit agent in an organic solvent which is miscible in water; and (b) addition of water to the mixture of step (a), wherein the amount of water is at a weight ratio between 10:1 to 1:5 based on the amount of the organic solvent.

In a third aspect, the present invention is directed to a home or personal care composition comprising microcapsules of the present invention.

All other aspects of the present invention will more readily become apparent upon considering the detailed description and examples which follow.

### Detailed description of the invention

Except in the examples, or where otherwise explicitly indicated, all numbers in this description indicating amounts of material or conditions of reaction, physical properties of materials and/or use may optionally be understood as modified by the word "about".

All amounts are by weight of the composition, unless otherwise specified.

It should be noted that in specifying any range of values, any particular upper value can be associated with any particular lower value.

For the avoidance of doubt, the word "comprising" is intended to mean "including" but not necessarily "consisting of" or "composed of". In other words, the listed steps or options need not be exhaustive.

The disclosure of the invention as found herein is to be considered to cover all embodiments as found in the claims as being multiply dependent upon each other irrespective of the fact that claims may be found without multiple dependency or redundancy.

"Aspect ratio" as used herein refers to the ratio of the length to width of the microcapsule. Both length and width may be measured by optical microscopy, for example Leica DM2500P. "Length" as used herein refers to the longest measurable distance of the microcapsule in the optical image. "Width" as used herein refers to the longest measurable distance of the microcapsule along the direction perpendicular to the length of the microcapsule in the optical image.

"Water-solvable" as used herein refers to a material that dissolves in water to give a solution with a concentration of at least 1 gram per litre. "Water-insolvable" as used herein refers to a material that dissolves in water to give a solution with a concentration of less than 1 gram per litre.

The microcapsules may have any non-spherical shape, for example elongated or rod-like or ellipsoid-like. It is preferable that the particle is ellipsoid or rod in shape and more preferably rod in shape. The microcapsule preferably has an aspect ratio of from 3:1 to 9:1, more preferably from 3:1 to 7:1, even more preferably from 4:1 to 7:1, and still even more preferably from 4.5:1 to 6.5:1.

The microcapsule has an average length of 0.5 to 100 µm, more preferably from 1 to 65 µm, even more preferably from 3 to 50 µm, still even more preferably from 7 to 35 µm and most preferably from 12 to 25 µm.

The water-insolvable solid particle comprises cellulose, modified cellulose or a mixture thereof. Most preferably the water-insolvable solid particle is ethyl cellulose particle.

The degree of substitution of the ethyl cellulose is preferably 2 to 3, more preferably 2.2 to 2.8. The degree of substitution refers to the average number of the hydroxyl group substituted per anhydroglucose unit (the 'monomer'). The maximum theoretical number of degree of substitution is 3 if all three hydroxyls are replaced.

Suitable cellulose (preferably ethyl cellulose) preferably has a dynamic viscosity of 5 to 300 mPa*s (5 to 300 cP) at a concentration of 5 wt% in tolune/ethanol (80:20 by weight), more preferably between 30 to 230 mPa*s (30 to 230 cP), and even more preferably 60 to 150 cP at such conditions.

Preferably the water-insolvable particle is ellipsoid or rod in shape and more preferably rod in shape. The particle preferably has an average length of 0.5 to 100 µm, more preferably from 1 to 65 µm, even more preferably from 3 to 50 µm, still even more preferably from 7 to 35 µm and most preferably from 12 to 25 µm.

Preferably the weight ratio of the water-insolvable particle to the benefit agent is 1:4 to 20:1, more preferably 1:2.5 to 10:1, and even more preferably 1:1.5 to 5:1.

Preferably, the microcapsule comprises one single water-insolvable solid particle. The benefit agent is distributed throughout the particle. The water-insolvable solid particle comprises a plurality of pores throughout the particles and the benefit is encapsulated inside the pores. Preferably the pore has a diameter of 5 to 100 nm, more preferably from 10 to 50 nm. "Diameter" of pore refers to the largest measurable distance of the pore. The diameter may be measured for example by scanning electron microscopy (SEM) by averaging the values of at least 10 pores.

The benefit agent is fragrance, pro-fragrance or a mixture thereof. Most preferably the benefit agent is fragrance.

Typically, the fragrance comprises components having a boiling point of less than 300, more preferably 100-250 Celsius, measured at one atmosphere. It is also advantageous to comprises components which have a LogP of less than 3.0 (i.e. those which will be partitioned into water).

The pro-fragrance can, for example, be a food lipid. Food lipids typically contain structural units with pronounced hydrophobicity. The majority of lipids are derived from fatty acids. In these 'acyl' lipids the fatty acids are predominantly present as esters and include mono-, di-, triacyl glycerols, phospholipids, glycolipids, diol lipids, waxes, sterol esters and tocopherols.

The fragrance is typically present in an amount of from 10 to85% by total weight of the particle, preferably from 15 to 75% by total weight of the particle. The fragrance suitably has a molecular weight of from 50 to 500 Dalton. Pro-fragrances can be of higher molecular weight, being typically 1 to 10k Dalton.

The microcapsule may be prepared in any suitable process. However, the process of the invention comprises steps of (a) dissolving the water-insolvable solid and benefit agent in an organic solvent which is miscible in water; and (b) addition of water to the mixture of step (a), wherein the amount of water is at a weight ratio between 1:5 to 10:1 based on the amount of the organic solvent.

More preferably, the process comprises step (c) of removing the organic solvent. Preferably, the process comprises step (d) of removing water to a concentration of microcapsule of at least 1% by weight.

Preferably, the weight ratio of the water-insolvable solid to the organic solvent is at least 1:70, more preferably from 1:50 to 1:1 and even more preferably from 1:30 to 1:5. The weight ratio of organic solvent to water in the mixture generated in step (b) is preferably 1:2 to 5:1 and more preferably from 1:1 to 2:1. Preferred organic solvent are ethanol, acetone, isopropanol, tetrahydrofuran, or a mixture thereof. More preferably, the organic solvent comprises ethanol.

It is preferable to add a water-soluble thickener in step (b). Preferably, the water-soluble thickener comprises gum, starch derivative, cellulose derivatives, carboxyvinyl polymer, or a mixture thereof. More preferably the water-soluble thickener is a cellulose derivative, and even more preferably a hydroxypropyl cellulose.

The end-product compositions of the invention may be in any physical form but preferably an aqueous-based liquid. The benefit agent delivery particle of the invention may be advantageously incorporated into personal care or home care compositions but preferably a personal care composition. The home care composition is preferably an aqueous laundry detergent or an aqueous fabric conditioner. The composition is preferably a skin cleansing composition containing a cleansing surfactant.

Typically, the composition comprises the particles at levels of from 0.001% to 10%, more preferably from 0.005% to 7.55%, even more preferably from 0.01 to 5%, and most preferably from 0.1% to 2% by weight of the total composition.

The composition preferably comprises a cleansing surfactant. More than one cleansing surfactant may be included in the composition. The cleaning surfactant may be chosen from soap, non-soap anionic, cationic, non-ionic, amphoteric surfactant and mixtures thereof. Many suitable surface-active compounds are available and are fully described in the literature, for example, in "Surface-Active Agents and Detergents", Volumes I and II, by Schwartz, Perry and Berch. The preferred surface-active compounds that can be used are soaps, non-soap anionic, non-ionic surfactant, amphoteric surfactant or a mixture thereof.

Suitable non-soap anionic surfactants include linear alkylbenzene sulphonate, primary and secondary alkyl sulphates, particularly C₈ to C₁₅ primary alkyl sulphates; alkyl ether sulphates; olefin sulphonates; alkyl xylene sulphonates; dialkyl sulphosuccinates; fatty acid ester sulphonates; or a mixture thereof. Sodium salts are generally preferred.

Most preferred non-soap anionic surfactant are linear alkylbenzene sulphonate, particularly linear alkylbenzene sulphonates having an alkyl chain length of from C₈ to C₁₅. It is preferred if the level of linear alkylbenzene sulphonate is from 0 wt% to 30 wt%, more preferably from 1 wt% to 25 wt%, most preferably from 2 wt% to 15 wt%, by weight of the total composition.

Nonionic surfactants that may be used include the primary and secondary alcohol ethoxylates, especially the C₈ to C₂₀ aliphatic alcohols ethoxylated with an average of from 1 to 20 moles of ethylene oxide per mole of alcohol, and more especially the C₁₀ to C₁₅ primary and secondary aliphatic alcohols ethoxylated with an average of from 1 to 10 moles of ethylene oxide per mole of alcohol. Non ethoxylated nonionic surfactants include alkylpolyglycosides, glycerol monoethers, and polyhydroxyamides (glucamide). It is preferred if the level of non-ionic surfactant is from 0 wt% to 30 wt%, preferably from 1 wt% to 25 wt%, most preferably from 2 wt% to 15 wt%, by weight of a fully formulated composition comprising the microcapsules of the invention.

Suitable amphoteric surfactants preferably are betaine surfactants. Examples of suitable amphoteric surfactants include, but are not limited to, alkyl betaines, alkylamido betaines, alkyl sulfobetaines, alkyl sultaines and alkylamido sultaines; preferably, those having 8 to about 18 carbons in the alkyl and acyl group. It is preferred that the amount of the amphoteric surfactant is 0 to 20 wt%, more preferably from 1 to 10 wt%, by weight of the composition.

It is also possible to include certain mono-alkyl cationic surfactants. Cationic surfactants that may be used include quaternary ammonium salts of the general formula R¹R²R³R⁴N⁺ X⁻ wherein the R groups are long or short hydrocarbon chains, typically alkyl, hydroxyalkyl or ethoxylated alkyl groups, and X is a counter-ion (for example, compounds in which R¹ is a C8-C22 alkyl group, preferably a C₈-C₁₀ or C12-C14 alkyl group, R² is a methyl group, and R³ and R⁴, which may be the same or different, are methyl or hydroxyethyl groups); and cationic esters (for example, choline esters).

Water-soluble skin benefit agents may optionally be formulated into the compositions of the invention. A variety of water-soluble skin benefit agents can be used and the level can be from 0.1 to 50% but preferably from 1 to 30% by weight of the composition. These materials include, but are not limited to, polyhydroxy alcohols. Preferred water-soluble skin benefit agents are glycerin, sorbitol and polyethylene glycol.

Water-insoluble skin benefit agents may also be formulated into the compositions as conditioners and moisturizers. Examples include silicone oils; hydrocarbons such as liquid paraffins, petrolatum, microcrystalline wax, and mineral oil; and vegetable triglycerides such as sunflower seed and cottonseed oils.

Some compositions may include thickeners. These may be selected from cellulosics, natural gums and acrylic polymers but not limited by this thickening agent types. Among the cellulosics are sodium carboxymethyl cellulose, hydroxyethyl cellulose, hydroxypropyl methylcellulose and combinations thereof. Suitable gums include xanthan, pectin, karaya, agar, alginate gums and combinations thereof. Among the acrylic thickeners are homopolymers and copolymers of acrylic and methacrylic acids including carbomers such as Carbopol 1382, Carbopol 982, Ultrez, Aqua SF-1 and Aqua SF-2 available from the Lubrizol Corporation. Amounts of thickener may range from 0.01 to 3% by weight of the active polymer (outside of solvent or water) in the compositions.

Preservatives can desirably be incorporated into the compositions of this invention to protect against the growth of potentially harmful microorganisms. Suitable traditional preservatives for compositions of this invention are alkyl esters of para-hydroxybenzoic acid. Other preservatives which have more recently come into use include hydantoin derivatives, propionate salts, and a variety of quaternary ammonium compounds. Particularly preferred preservatives are phenoxyethanol, methyl paraben, propyl paraben, imidazolidinyl urea, sodium dehydroacetate and benzyl alcohol. The preservatives should be selected having regard for the use of the composition and possible incompatabilities between the preservatives and other ingredients. Preservatives are preferably employed in amounts ranging from 0.01% to 2% by weight of the composition.

A variety of other optional materials may be formulated into the compositions. These may include: antimicrobials such as 2-hydroxy-4,2',4'-trichlorodiphenylether (triclosan), 2,6-dimethyl-4-hydroxychlorobenzene, and 3,4,4'-trichlorocarbanilide; scrub and exfoliating particles such as polyethylene and silica or alumina; cooling agents such as menthol; skin calming agents such as aloe vera; and colorants.

In addition, the compositions of the invention may further include 0.5 to 10% by weight of sequestering agents, such as tetra sodium ethylenediaminetetraacetate (EDTA), EHDP or mixtures; opacifiers and pearlizers such as ethylene glycol distearate, titanium dioxide or Lytron 621 (Styrene/Acrylate copolymer); all of which are useful in enhancing the appearance or properties of the product.

Preferably the composition comprises water in an amount of at least 5% by weight of the composition, more preferably at least 25%, even more preferably 40 to 90% and still even more preferably at least 60 to 85% by weight of the composition.

The following examples are provided to facilitate an understanding of the invention. The examples are not intended to limit the scope of the claims.

### Examples

### Materials

| Name | Abbreviation | Description | Supplier |
|---|---|---|---|
| Ethyl cellulose | EC | Aqualon™ N-100 | Ashland |
| Hydropropyl cellulose | HPC | Product number: H0475 | TCI |
| Fluorescence yellow 3G | FY3G | - | Shanghai Qinba Chemie Co., Ltd. |
| Damascone | - | Damascone Delta | Beijing Peking University Zoteq Co., Ltd. |

### Example 1

This example demonstrates the preparation and characterization of the microcapsules.

### (1) Preparation of EC microcapsule

50 mg of FY3G was dispersed in 20 g of Damascone, followed by sonication for 25 minutes to form a mixture. 0.96 g of EC powder was dissolved in 24 g of ethanol by shearing at 2000 rpm for 20 minutes, followed by adding 0.28 g of the mixture to get a EC solution. 0.24 g of HPC was dissolved in 20 g of ethanol and 28 g of water by shearing at 2000 rpm for 3 minutes to get a HPC solution. Then, the EC solution was added into the HPC solution quickly with homogenizing at 16,000 rpm for 2 minutes to get a EC microcapsule mixture. The ethanol and HPC was removed by repeating washing EC microcapsule mixture by water via centrifugation for three times. An EC microcapsule dispersion containing 10 wt% of EC microcapsule (named as microcapsule 1) was obtained.

Other microcapsules were prepared in a similar manner as described above but the raw materials for preparing the microcapsule were in accordance with Table 1.

**Table 1**

| | EC solution | HPC solution | | | | |
|---|---|---|---|---|---|---|
| Microcapsule | EC (g) | Ethanol (g) | HPC (g) | Water (g) | Ethanol (g) | Damascone (g) |
| 2 | 2.8 | 28 | 0.66 | 22 | 0 | 0.4 |
| 3 | 2.8 | 28 | 0.66 | 22 | 0 | 0.2 |
| 4 | 0.96 | 24 | 0.24 | 36 | 12 | 0.28 |
| 5 | 2.71 | 27 | 0.11 | 21.5 | 0 | 0* |

| | | | | | | |
|---|---|---|---|---|---|---|
| *1mg of fluorescence yellow 3G was added. | | | | | | |

### (2) Characterization of the microcapsule

Optical microscopy (DM2500P, Leica, Germany) equipped with a fluorescence light was used to observe the morphology. The length (L) and weight (W) were obtained and the aspect ratios were calculated by L/W. The average aspect ratios were calculated by averaging the values for at least 10 microcapsules in the image.

**Table 2**

| Microcapsule | Average Length(µm) | Average Aspect Ratio |
|---|---|---|
| 1 | 8.0 | 1.0 |
| 2 | 11.2 | 2.8 |
| 3 | 13.2 | 3.2 |
| 4 | 16.5 | 5.5 |
| 5 | 30.0 | 15.0 |

Microcapsules 1, 2 and 5 are comparative examples.

Fluorescence imaging demonstrated that the fluorescence agent was distributed evenly throughout the particle which indicates that the fragrance (damascone) is distributed evenly throughout the microcapsule.

### Example 2

This example demonstrates the deposition efficiency of the microcapsules.

A typical skin wash procedure is shown as follows. A pig skin piece (2 cm x 2 cm) was placed in a Petri dish, to which 25 mg of formulation in Table 3 was added together with 33 µL of water dispersion of microcapsule (10 wt%) as prepared in Example 1. The skin, with the mixture onto it, was then gently rubbed with another piece of skin for 30 seconds and the two pieces were rinsed with total 250 mL of tap water. The two pieces of skin were then immersed in a vial containing 10 ml of ethanol for one hour. 200 µL of the extraction liquid (ethanol) was withdrawn from the vial and added to a 96-well microplate for fluorescence measurement (excitation: 480 nm, emission: 520 nm) to afford a reading of E₁.

**Table 3**

| Ingredient | Wt% |
|---|---|
| Sodium Laureth Sulfate | 12.86 |
| Cocamidopropyl Betaine | 5.67 |
| Cocamide MEA | 1.35 |
| Acrylates Copolymer | 6.00 |
| Tetrasodium EDTA | 0.13 |
| Citric Acid Monohydrate | 0.10 |
| Sodium Hydroxide | 0.20 |
| Polypropylene glycol-400 | 0.70 |
| Sodium Chloride | 0.15 |
| Iodopropynyl Butylcarbamate | 0.07 |
| Phenoxyethanol | 0.60 |
| Water | to 100 |

Another two skin pieces were placed in a Petri dish, to which 33 µL of water dispersion of microcapsules (10 wt%) was added. The skin pieces with the microcapsule dispersion were immediately immersed in a vial containing 10 mL of ethanol for one hour. 200 µL of the extraction liquid was withdrawn from the vial and added to a 96-well microplate for fluorescence measurement (excitation: 480 nm, emission 520: nm) to afford a reading of E₀.

In addition, two skin pieces were immersed in a vial containing 10 mL of ethanol for one hour. 200 µL of the extraction liquid was withdrawn from the vial and added to a 96-well microplate for fluorescence measurement (excitation: 480 nm, emission: 520 nm) to afford a reading of E_{b}.

The deposition efficiency was calculated according to the following equation: Deposition efficiency = (E₁-E_{b}) / (E₀-E_{b}) x 100%. The deposition efficiencies for the microcapsules were listed in Table 4.

**Table 4**

| Microcapsule | Aspect Ratio | Deposition efficiency (%) |
|---|---|---|
| 1 | 1.0 | 5.4 ± 0.9 |
| 2 | 2.8 | 3.9 ± 1.2 |
| 3 | 3.2 | 6.9 ± 0.8 |
| 4 | 5.5 | 13.9 ± 7.7 |
| 5 | 15.0 | 4.8 ± 0.4 |

## Claims

1. A microcapsule comprising benefit agent and water-insolvable solid particle, wherein the microcapsule has an aspect ratio of from 3:1 to 12:1 and the weight ratio of the particle to the benefit agent is 1:5 to 50:1, where aspect ratio means the ratio of the length to width of the microcapsule as measured by optical microscopy, where length refers to the longest measurable distance of the microcapsule in the optical image and width refers to the longest measurable distance of the microcapsule along the direction perpendicular to the length of the microcapsule in the optical image, and where water-insolvable refers to a material that dissolves in water to give a solution with a concentration of less than 1 gram per litre,
wherein,
the water-insolvable solid particle comprises a plurality of pores throughout the particles and the benefit agent is encapsulated inside the pores; wherein the particle comprises cellulose; wherein the benefit agent is fragrance, pro-fragrance, or a mixture thereof.

2. The microcapsule according to claim 1 wherein the benefit agent is distributed throughout the particle.

3. The microcapsule according to claim 2 wherein the pore has a diameter of 5 to 100 nm, preferably from 10 to 50 nm.

4. The microcapsule according to any one of the preceding claims wherein the particle comprises ethyl cellulose.

5. The microcapsule according to any one of the preceding claims wherein the particle is rod in shape.

6. The microcapsule according to any one of the preceding claims microcapsule has an aspect ratio of from 3:1 to 9:1, preferably from 3:1 to 7:1, more preferably from 4:1 to 7:1, and even more preferably from 4.5:1 to 6.5:1.

7. The microcapsule according to any one of the preceding claims wherein the particle has an average length of 0.5 to 100 microns, preferably from 1 to 65 microns.

8. The microcapsule according to any one of the preceding claims wherein the benefit agent is fragrance.

9. The microcapsule according to any one of the preceding claims wherein the weight ratio of the particle to the benefit agent is from 30:1 to 1:2.

10. A process for preparing a microcapsule according to any one of claims 1 to 9 comprising
steps of:
a) dissolving the water-insolvable solid and benefit agent in an organic solvent which is miscible in water; and,
b) addition of water to the mixture of step (a), wherein the amount of water is at a weight ratio between 10:1 to 1:5 based on the amount of the organic solvent.

11. A home or personal care composition comprising microcapsules of any one of the preceding claims, preferably the microcapsule is present in amount of 0.01 to 2% by weight of the composition.

12. The composition according to claim 11 wherein the composition comprises a cleansing surfactant.

13. The composition according to claim 11 or 12 wherein the composition is a skin cleansing composition.

## Patentansprüche

1. Mikrokapsel, umfassend Vorteilsmittel und wasserunlösliches festes Teilchen, wobei die Mikrokapsel ein Aspektverhältnis von 3:1 bis 12:1 aufweist und das Gewichtsverhältnis des Teilchens zum Vorteilsmittel 1:5 bis 50:1 beträgt, wobei Aspektverhältnis das Verhältnis der Länge zur Breite der Mikrokapsel bedeutet, gemessen durch optische Mikroskopie, wobei sich die Länge auf den längsten messbaren Abstand der Mikrokapsel im optischen Bild bezieht und die Breite sich auf den längsten messbaren Abstand der Mikrokapsel entlang der Richtung senkrecht zur Länge der Mikrokapsel im optischen Bild bezieht, und wobei sich wasserunlöslich auf ein Material bezieht, das sich in Wasser löst, um eine Lösung mit einer Konzentration von weniger als 1 Gramm pro Liter zu ergeben;
wobei,
das wasserunlösliche feste Teilchen eine Mehrzahl von Poren in den Teilchen umfasst und das Vorteilsmittel in den Poren gekapselt ist; wobei das Teilchen Cellulose umfasst; wobei das Vorteilsmittel Duftstoff, Duftstoffvorstufe oder eine Mischung davon ist.

2. Mikrokapsel nach Anspruch 1, wobei das Vorteilsmittel über das ganze Teilchen verteilt ist.

3. Mikrokapsel nach Anspruch 2, wobei die Pore einen Durchmesser von 5 bis 100 nm, bevorzugt von 10 bis 50 nm, aufweist.

4. Mikrokapsel nach irgendeinem der vorhergehenden Ansprüche, wobei das Teilchen Ethylcellulose umfasst.

5. Mikrokapsel nach irgendeinem der vorhergehenden Ansprüche, wobei das Teilchen eine Stabform aufweist.

6. Mikrokapsel nach irgendeinem der vorhergehenden Ansprüche, wobei die Mikrokapsel ein Aspektverhältnis von 3:1 bis 9:1, bevorzugt von 3:1 bis 7:1, bevorzugter von 4:1 bis 7:1 und noch bevorzugter von 4,5:1 bis 6,5:1, aufweist.

7. Mikrokapsel nach irgendeinem der vorhergehenden Ansprüche, wobei das Teilchen eine durchschnittliche Länge von 0,5 bis 100 Mikron, bevorzugt von 1 bis 65 Mikron, aufweist.

8. Mikrokapsel nach irgendeinem der vorhergehenden Ansprüche, wobei das Vorteilsmittel Duftstoff ist.

9. Mikrokapsel nach irgendeinem der vorhergehenden Ansprüche, wobei das Gewichtsverhältnis des Teilchens zum Vorteilsmittel 30:1 bis 1:2 beträgt.

10. Verfahren zur Herstellung einer Mikrokapsel nach irgendeinem der Ansprüche 1 bis 9, umfassend die Schritte:
a) Lösen des wasserunlöslichen Feststoffs und des Vorteilsmittels in einem organischen Lösungsmittel, das mit Wasser mischbar ist; und
b) Zugabe von Wasser zu der Mischung von Schritt (a), wobei die Menge an Wasser in einem Gewichtsverhältnis zwischen 10:1 bis 1:5, bezogen auf die Menge des organischen Lösungsmittels, vorliegt.

11. Haushalts- oder Körperpflegezusammensetzung, umfassend Mikrokapseln nach irgendeinem der vorhergehenden Ansprüche, wobei die Mikrokapsel bevorzugt in einer Menge von 0,01 bis 2 Gew.-% der Zusammensetzung vorliegt.

12. Zusammensetzung nach Anspruch 11, wobei die Zusammensetzung ein Reinigungstensid umfasst.

13. Zusammensetzung nach Anspruch 11 oder 12, wobei die Zusammensetzung eine Hautreinigungszusammensetzung ist.

## Revendications

1. Microcapsule comprenant un agent bénéfique et une particule solide insoluble dans l'eau, dans laquelle la microcapsule présente un rapport des dimensions de 3:1 à 12:1 et le rapport massique de la particule à l'agent bénéfique est de 1:5 à 50:1, où le rapport des dimensions indique le rapport de la longueur à largeur de la microcapsule comme mesuré par microscopie optique, où la longueur fait référence à la distance mesurable la plus longue de la microcapsule dans l'image optique et la largeur fait référence à la distance mesurable la plus longue de la microcapsule le long de la direction perpendiculaire à la longueur de la microcapsule dans l'image optique, et où insoluble dans l'eau fait référence à un matériau qui se dissout dans l'eau pour fournir une solution avec une concentration inférieure à 1 gramme par litre,
dans laquelle,
la particule solide insoluble dans l'eau comprend plusieurs pores dans la totalité des particules et l'agent bénéfique est encapsulé à l'intérieur des pores ; dans laquelle la particule comprend de la cellulose ; dans laquelle l'agent bénéfique est un parfum, pro-parfum, ou un mélange de ceux-ci.

2. Microcapsule selon la revendication 1, dans laquelle l'agent bénéfique est distribué dans la totalité de la particule.

3. Microcapsule selon la revendication 2, dans laquelle le pore présente un diamètre de 5 à 100 nm, de préférence de 10 à 50 nm.

4. Microcapsule selon l'une quelconque des revendications précédentes, dans laquelle la particule comprend de l'éthylcellulose.

5. Microcapsule selon l'une quelconque des revendications précédentes, dans laquelle la partie est en forme de tige.

6. Microcapsule selon l'une quelconque des revendications précédentes, dans laquelle la microcapsule présente un rapport des dimensions de 3:1 à 9:1, de préférence de 3:1 à 7:1, encore mieux de 4:1 à 7:1, et bien mieux encore de 4,5:1 à 6,5:1.

7. Microcapsule selon l'une quelconque des revendications précédentes, dans laquelle la particule présente une longueur moyenne de 0,5 à 100 microns, de préférence de 1 à 65 microns.

8. Microcapsule selon l'une quelconque des revendications précédentes, dans laquelle l'agent bénéfique est un parfum.

9. Microcapsule selon l'une quelconque des revendications précédentes, dans laquelle le rapport massique de la particule à l'agent bénéfique est de 30:1 à 1:2.

10. Procédé pour la préparation d'une microcapsule selon l'une quelconque des revendications 1 à 9 comprenant les étapes de :
a) dissolution des solide insoluble dans l'eau et agent bénéfique dans un solvant organique qui est miscible dans l'eau ; et,
b) addition d'eau au mélange de l'étape (a), dans lequel la quantité d'eau est à un rapport massique de 10:1 à 1:5 sur la base de la quantité du solvant organique.

11. Composition pour l'hygiène de la maison ou de la personne comprenant des microcapsules selon l'une quelconque des revendications précédentes, la microcapsule est de préférence présente dans une quantité de 0,01 à 2 % en masse de la composition.

12. Composition selon la revendication 11, dans laquelle la composition comprend un tensioactif nettoyant.

13. Composition selon la revendication 11 ou 12, dans laquelle la composition est une composition pour le nettoyage de la peau.
